**Europäisches Patentamt**

**European Patent Office**

**Office européen des brevets**

(19)

(11) Veröffentlichungsnummer: **0 114 414**
**B1**

(12)

# EUROPÄISCHE PATENTSCHRIFT

(45) Veröffentlichungstag der Patentschrift:
**01.06.88**

(51) Int. Cl.⁴: **A 61 K 7/09**

(21) Anmeldenummer: **83113206.3**

(22) Anmeldetag: **29.12.83**

(54) **Verfahren zur Dauerverformung des Haarnachwuchses und Mittel zu seiner Durchführung.**

(30) Priorität: **19.01.83 DE 3301515**

(43) Veröffentlichungstag der Anmeldung:
**01.08.84 Patentblatt 84/31**

(45) Bekanntmachung des Hinweises auf die Patenterteilung:
**01.06.88 Patentblatt 88/22**

(84) Benannte Vertragsstaaten:
**AT BE CH DE FR GB IT LI NL SE**

(56) Entgegenhaltungen:
**EP - A - 0 059 428**
**DE - B - 1 239 062**

**PATENTS ABSTRACTS OF JAPAN, Band 4, Nr.**
**93(C-17)(575), 5. Juli 1980, Seite 18C17**

(73) Patentinhaber: **Wella Aktiengesellschaft, Berliner Allee 65, D-6100 Darmstadt (DE)**

(72) Erfinder: **Hoch, Dietrich, Ringstrasse 48, D-6102 Pfungstadt (DE)**
Erfinder: **Wajaroff, Theodor, Erbacher Strasse 56b, D-6100 Darmstadt (DE)**
Erfinder: **Konrad, Eugen, Mecklenburger Strasse 101, D-6100 Darmstadt (DE)**

ACTORUM AG

### Beschreibung

Die dauerhafte Haarverformung erfolgt bei den zur Zeit üblichen Verfahren in zwei Stufen. Zunächst werden durch die Einwirkung eines geeigneten Reduktionsmittels die Disulfidbrücken des Haarkeratins gespalten. Das Haar wird sodann in eine neue Form gebracht und anschliessend durch die Behandlung mit einem geeigneten Oxidationsmittel, unter Wiederverknüpfung der gespaltenen Disulfidbindungen, in der neuen Form fixiert.

Die zur Durchführung der ersten, reduzierenden Verfahrensstufe verwendeten Mittel enthalten als verformungswirksame keratinreduzierende Substanz Sulfit oder bestimmte Mercaptoverbindungen, insbesondere Thioglykolsäure, Thiomilchsäure, auch in Form ihrer Salze mit anorganischen Basen oder auch Thioglycerin bzw. Derivate dieser Mercaptoverbindungen. In der Regel sind diese Mittel alkalisch eingestellt, um eine Haarerweichung und damit ein rasches Eindringen der keratinreduzierenden Substanz in das Haarkeratin zu erreichen. Die erforderliche Alkalität wird hiebei vor allem durch die Zugabe von Ammoniak, organischen Aminen, Ammonium- und Alkalicarbonat, Ammonium- und Alkalihydrogencarbonat erreicht. Die praktische Durchführung der dauerhaften Verformung von menschlichen Haaren erfolgt im allgemeinen in der Weise, dass man das gewaschene und handtuchtrockene Haar zunächst in mehrere Partien aufteilt und diese Partien sodann auf Wickler wickelt. Nach Beendigung des Wickelvorganges werden die Wickler mit der dafür erforderlichen Menge des Dauerverformungsmittels gründlich durchgefeuchtet. Die für eine Dauerwellung verwendeten Wickler haben einen Durchmesser von 5 bis 13 Millimeter, während für eine Haarentkräuselung Wickler mit einem Durchmesser von über 13 Millimeter erforderlich sind.

Die Einwirkungszeit des Haarverformungsmittels auf das Haar beträgt sowohl bei der Dauerwellung als auch bei der dauerhaften Entkräuselung, je nach Haarbeschaffenheit und dem gewünschten Grad der Umformung, im allgemeinen bis zu 30 Minuten. Durch Wärmezufuhr, beispielsweise unter Verwendung eines Wärmestrahlers oder einer Trockenhaube, lässt sich die Einwirkungszeit verkürzen.

Nach Ablauf der erforderlichen Einwirkungszeit des Haarverformungsmittels wird das gewickelte Haar mit Wasser gespült und mit einer wässrigen Lösung eines Oxidationsmittels, vorzugsweise mit einer 2%igen Hydrogenperoxidlösung, unter Wiederverknüpfung der zuvor gespaltenen Disulfidbindungen des Haarkeratins in der neuen Form fixiert.

Die Einwirkungszeit des Fixiermittels beträgt hierbei üblicherweise 10 bis 15 Minuten. Schliesslich werden die Wickler entfernt und das Haar gründlich mit Wasser ausgespült.

Da das Haar im Monat 1 bis 2 cm wächst, muss die Dauerverformung nach 3–4 Monaten wiederholt werden. Oft ist jedoch das durch die letzte Dauerverformung bereits verformte Haar bezüglich der Umformung noch in ausreichend gutem Zustand, so dass eine selektive Umformung des in einer Länge von 3 bis 8 cm nachgewachsenen Haarnachwuchses für die Wiederherstellung der Frisur ausreichen würde.

Bisher gibt es jedoch kein praktikables Verfahren für eine getrennte Behandlung des unverformten Haarnachwuchses und der noch ausreichend verformten Haarlänge. Dehnt man die Verformungsbehandlung jedoch auf die Gesamtlänge aus, so wird der bereits verformte Haarteil chemisch besonders stark beansprucht, und zwar zur Haarspitze hin in zunehmendem Masse. Eine Haarschädigung ergibt sich nicht alleine dadurch, dass das Haar zur Spitze hin bereits mehrmals verformt wurde, sondern hauptsächlich deswegen, weil es dort länger den Umwelteinflüssen (z.B. Sonne) ausgesetzt und mechanisch (z.B. durch Kämmen) stärker beansprucht war.

Aus der deutschen Offenlegungsschrift 1 467 853 ist es bekannt, das Haar vor dem Wickeln auf Dauerwellwickler mit einem flüssigen Vorneutralisierungsmittel, das ein Oxidationsmittel enthält und das gegebenenfalls zusätzlich auf einen pH-Wert im sauren Bereich eingestellt ist, zu befeuchten. Eine selektive Behandlung des nachgewachsenen Haares ist hierbei jedoch nicht erreichbar, da die Dauerwellflüssigkeit den gesamten Wickler durchdringt. Ausserdem werden die besonders strapazierten und empfindlichen Haarspitzen längere Zeit der Einwirkung des Oxidationsmittels ausgesetzt und so ebenfalls geschädigt. Die Verwendung von sogenanntem Spitzenpapier (dabei werden die Haarspitzen zwischen ein gefaltetes Blatt, welches zusätzlich mit haarschützenden beziehungsweise haarpflegenden Stoffen imprägniert sein kann, gelegt) ist umständlich und zeitraubend. Zudem ist hierdurch nur ein Schutz der Haarspitzen, nicht aber der übrigen bereits verformten Haarlänge möglich.

Es bestand daher die Aufgabe, ein Verfahren zur alleinigen Umformung des Haarnachwuchses ohne wesentliche Einwirkung des reduktiven Dauerverformungsmittels auf die bereits verformten Haarbereiche zu finden.

Hierzu wurde nun gefunden, dass durch ein Verfahren zur selektiven Dauerverformung des Haarnachwuchses, bei dem man das Haar zunächst mit einem flüssigen wässrigen Vorbehandlungsmittel behandelt, auf Wickler wickelt, mit einem wässrigen keratinreduzierenden Dauerverformungsmittel auf der Basis von Sulfit oder Mercaptoverbindungen behandelt, spült, oxidativ fixiert und in üblicher Weise weiterbehandelt, dadurch gekennzeichnet, dass man als Vorbehandlungsmittel eine flüssige wässrige Zusammensetzung verwendet, welche mindestens ein physiologisch verträgliches organisches Salz, anorganisches Salz, natürliches Polycarbonsäurepolymer oder synthetisches Polycarbonsäurepolymer enthält, das bei einem pH-Wert von 2 bis 7 in wässriger Lösung löslich oder dispergierbar ist und bei einem pH-Wert von 7,5 bis 11 ein Gel oder eine Ausfällung ergibt, und sodann ein Dauerverformungsmittel mit einem pH-Wert von 7,5 bis 11 verwendet, die gestellte Aufgabe in hervorragender Weise gelöst wird.

Das reduzierende Dauerverformungsmittel dringt in die oberste Lage des gewickelten Haares ein und bildet durch Reaktion mit dem Vorbehandlungsmittel sofort eine Trennschicht in Form eines Gels oder einer Ausfällung aus, welche ein weiteres Eindringen des Dauerverformungsmittels in tiefere Lagen des gewickelten Haares erschwert oder verhindert. Es ist somit eine selektive Umformung des die oberste Lage des Wicklers bildenden Haarnachwuchses möglich.

Beispiele für geeignete physiologisch verträgliche, organische oder anorganische Salze sind Aluminiumacetat, Ammoniumaluminiumsulfat und Ammoniumcernitrat. Es kommen auch Salze von makromolekularen Verbindungen in Betracht, sofern sie die oben genannten Voraussetzungen erfüllen. Beispielsweise können physiologisch verträgliche wasserlösliche Salze des Chitosans, wie z.B. Chitosanasparaginat, Chitosanlactat und Chitosanacetat, verwendet werden.

Als Polycarbonsäurepolymere, die in schwach saurer Lösung löslich oder dispergierbar sind und bei einem schwach alkalischen pH-Wert ein Gel oder eine Ausfällung ergeben, kommen besonders bevorzugt physiologisch verträgliche hochpolymere natürliche oder synthetisch Polycarbonsäurepolymere, das heisst makromolekulare Verbindungen, die entweder ganz oder teilweise aus eine freie Carbonsäuregruppe enthaltenen Monomereinheiten aufgebaut sind, in Betracht. Als natürliches Polycarbonsäurepolymer sei insbesondere Alginsäure genannt. Besonders geeignete synthetische Polymere, welche ganz aus Monomereinheiten mit einer freien Carbonsäuregruppe bestehen, sind beispielsweise physiologisch verträgliche hochpolymere Homopolymerisate der Acrylsäure und der Methacrylsäure. Beispiele für makromolekulare Verbindungen, welche lediglich teilweise aus Monomereinheiten mit einer freien Carbonsäuregruppe aufgebaut sind, sind hochpolymere Co- bzw. Mischpolymerisate aus Acrylsäure und/oder Methacrylsäure mit Acrylsäure- oder Methacrylsäureestern, Acrylsäure- oder Methacrylsäureamiden, Acrylsäure- oder Methacrylsäureimiden, Crotonsäure, Vinylacetat, Styrol oder anderen Vinyl- oder Allylderivaten, wobei mindestens 25 Mol-% des Polymerisates aus Carbonsäuregruppen enthaltenden Einheiten bestehen. Insbesondere kann das Vorbehandlungsmittel eine wässrige Dispersion aus einem hochmolekularen Copolymerisat von Acrylsäure/Methacrylsäure oder Acrylsäure/Acrylsäureester oder Acrylsäure/Methacrylsäureester oder Methacrylsäure/Acrylsäureester oder Methacrylsäure/Methacrylsäureester, wobei die genannten Ester insbesondere der Methyl-, Ethyl-, Propyl- oder Butylester sind, enthalten. Copolymerisate aus Methacrylsäure und Acrylsäureethylester werden beispielsweise von der Firma Röhm GmbH, Darmstadt, unter der Handelsbezeichnung ROHAGIT® vertrieben. Diese Polymerisate kommen sowohl in Pulverform (ROHAGIT S) als auch in Form wässriger Dispersionen (ROHAGIT SD 15) in den Handel.

Bei dem erfindungsgemässen Verfahren soll in dem Vorbehandlungsmittel das organische Salz, anorganische Salz, natürliche Polycarbonsäurepolymer oder synthetische Polycarbonsäurepolymer in einer Konzentration von 1 bis 10 Gew.%, bevorzugt 2 bis 6 Gew.%, Verwendung finden.

Die Ausbildung einer isolierenden gelförmigen Trennschicht zwischen Vorbehandlungsmittel und Dauerverformungsmittel kann leicht durch ein Experiment veranschaulicht werden, bei dem in einem Reagenzglas von 14 mm Durchmesser 5 cm³ einer klaren Dauerverformungsflüssigkeit A (aus Beispiel 1) vorsichtig, z.B. unter Verwendung einer Pipette, mit 5 cm³ eines klaren Vorbehandlungsmittels gemäss Beispiel 5 überschichtet werden. An der Phasengrenze bildet sich eine etwa 5 mm dicke trübe gelförmige Schicht aus, die eine Vermischung des Vorbehandlungsmittels mit dem Dauerwellmittel verhindert.

Die bisher geschilderte Ausführungsform des erfindungsgemässen Verfahrens beruht auf dem Prinzip, einen im Vorbehandlungsmittel enthaltenen Stoff infolge der Alkalität des Dauerverformungsmittels auszufällen oder zur Ausbildung eines Gels zu veranlassen. Das erfindungsgemässe Verfahren lässt sich jedoch auch mit verschiedenen anderen Kombinationen von Vorbehandlungsmitteln und Dauerverformungsmitteln durchführen. Voraussetzung ist lediglich, dass das Vorbehandlungsmittel beim Kontakt mit dem Dauerverformungsmittel eine Trennschicht ausbildet, welche ein weiteres Eindringen des Dauerverformungsmittels in die inneren Lagen des gewickelten Haares verhindert.

Das hier beschriebene Verfahren kann auch in der Weise durchgeführt werden, dass das Vorbehandlungsmittel und das Dauerverformungsmittel jeweils einen Stoff enthalten, welche so ausgewählt sind, dass sie in Kombination miteinander in wässriger Lösung, unabhängig vom pH-Wert der Lösung, ein Gel oder eine Ausfällung bilden. In diesem Falle ist es nicht erforderlich, dass der pH-Wert des verwendeten Dauerverformungsmittels im alkalischen Bereich liegt.

Eine weitere Ausführungsform der vorliegenden Erfindung ist daher ein Verfahren zur selektiven Dauerverformung des Haarnachwuchses, bei dem man das Haar zunächst mit einem flüssigen wässrigen Vorbehandlungsmittel behandelt, auf Wickler wickelt, mit einem wässrigen keratinreduzierenden Dauerverformungsmittel auf der Basis von Sulfit oder Mercaptoverbindungen behandelt, spült, oxidativ fixiert und in üblicher Weise weiterbehandelt, dadurch gekennzeichnet, dass man als Vorbehandlungsmittel eine wässrige Zusammensetzung verwendet, welche Alginsäure oder ein Alkalialginat enthält und sodann ein Dauerverformungsmittel verwendet, das ein wasserlösliches Salz des Calciums enthält.

Als Beispiel für ein wasserlösliches Salz des Calciums sei $Ca Cl_2$ genannt.

Beim Kontakt des Dauerverformungsmittels mit dem Vorbehandlungsmittel wird eine Trennschicht aus gelförmigen Calciumalginat gebildet.

Eine weitere Ausführungsform der Erfindung besteht in einem Verfahren zur selektiven Dauerverformung des Haarnachwuchses, bei dem man das Haar zunächst mit einem flüssigen wässrigen Vorbehandlungsmittel behandelt, auf Wickler wickelt, mit einem wässrigen keratinreduzierenden Dauerverformungsmittel auf der Basis von Sulfit oder Mercaptoverbindungen behandelt, spült, oxidativ fixiert und in üblicher Weise weiterbehandelt, dadurch gekennzeichnet, dass man als Vorbehandlungsmittel eine flüssige wässrige Zusammensetzung verwendet, welche ein anionisches Polymer enthält und sodann ein Dauerverformungsmittel verwendet, welches ein kationisches Polymer enthält.

Selbstverständlich können die beiden Komponenten ohne Nachteile vertauscht werden, so dass das anionische Polymer im Dauerverformungsmittel enthalten ist, während das kationische Polymer ein Bestandteil des Vorbehandlungsmittels ist.

Das kationische Polymer ergibt mit dem anionischen Polymer in wässriger Lösung eine Ausfällung, die in gewünschter Weise als Trennschicht wirkt.

Das anionische und das kationische Polymer sollen jeweils in einer Menge von 0,5 bis 3,0 Gew.% im Vorbehandlungsmittel beziehungsweise im Dauerverformungsmittel enthalten sein, wobei ein äquimolares Verhältnis zwar zweckmässig, jedoch nicht unbedingt erforderlich ist.

Geeignete anionische Polymere sind zum Beispiel Copolymerisate aus Crotonsäure, Vinylacetat und Acrylsäure.

Beispiele für geeignete kationische Polymere sind Dimethyldiallylammoniumchlorid-Homopolymer sowie Copolymerisate aus Vinylpyrrolidon und Dimethylaminoethylmethacrylat, welche mit Dimethylsulfat quaternisiert wurden.

Ausserdem ist es möglich, dass man die vorstehend genannten Ausführungsformen kombiniert, so dass zum Beispiel als Vorbehandlungsmittel eine saure Zusammensetzung verwendet wird, welche sowohl ein wasserlösliches Aluminiumsalz wie Ammoniumaluminiumsulfat (in schwach saurer Lösung löslich bildet im alkalischen Medium eine gelförmige Ausfällung von Aluminiumhydroxyd) als auch ein kationisches Polymer, wie zum Beispiel Dimethyldiallylammoniumchlorid-Homopolymerisat, enthält, und anschliessend ein alkalisches Dauerverformungsmittel mit einem Gehalt an einem anionischen Polymer verwendet. Ein weiteres Beispiel hierfür ist Beispiel 7. Beim Kontakt des Dauerverformungsmittels mit dem Vorbehandlungsmittel erfolgt die Ausbildung der Trennschicht auf zweierlei Weise; einmal durch das Aluminiumsalz in alkalischem Medium und zum anderen durch die Ausfällung, welche durch das anionische Polymer mit dem kationischen Polymer gebildet wird. Das Vorhandensein beider Stoffe in einem Vorbehandlungsmittel hat weiterhin den Vorteil, dass das Vorbehandlungsmittel sowohl in Kombination mit einem alkalischen Dauerverformungsmittel als auch in Kombination mit einem nicht alkalischen, jedoch ein anionisches Polymer enthaltenden Dauerverformungsmittel verwendet werden kann.

Obwohl das erfindungsgemässe Verfahren mit sehr gutem Ergebnis mit einem Vorbehandlungsmittel der vorstehend beschriebenen Art ausgeführt werden kann, das lediglich in wässriger Lösung, Emulsion oder Dispersion einen Stoff enthält, der beim Kontakt mit dem Dauerverformungsmittel ein Gel oder eine Ausfällung ergibt, so ist es doch besonders vorteilhaft, wenn man ein Vorbehandlungsmittel verwendet, das zusätzlich mindestens einen Stoff aus den nachfolgend aufgeführten Stoffgruppen a, b, c oder d enthält, welche ausserdem in der Lage sind, alkalische Dauerverformungsmittel zu neutralisieren, Reduktionsmittelreste aus dem Haar zu entfernen, die Quellung des Haares zu verhindern oder aber die eine pflegende Wirkung auf das Haar haben.

a) Das bei dem hier beschriebenen Verfahren verwendete Vorbehandlungsmittel kann zur Einstellung eines sauren pH-Wertes, insbesondere bei Verwendung eines alkalischen Dauerverformungsmittels, zusätzlich eine physiologisch verträgliche schwache Säure, wie zum Beispiel Zitronensäure, Weinsäure, Milchsäure, Essigsäure und Phosphorsäure, oder aber saure Phosphate enthalten. Das Vorbehandlungsmittel kann aber auch Puffersubstanzen, wie zum Beispiel neutrale oder saure Aminosäuren, enthalten.

b) Weiterhin kann das Vorbehandlungsmittel zur Eliminierung von Reduktionsmittelanteilen, welche eventuell während des Abspülens des Reduktionsmittels in das gewickelte Haar eindringen, zusätzlich Natriumbromat, Aconitsäure, Acetylendicarbonsäure, Ethylendicarbonsäure, Ethylmaleinsäure, α-Ethylcrotonsäure, i-Amylmaleinsäure, Angelicasäure, n-Butylfumarsäure, n- und i-Butylmaleinsäure, Citraconsäure, Crotonsäure, Fumarsäure, trans-Glutaconsäure, Isopropylmaleinsäure, Itaconsäure, Maleinsäure, Mesaconsäure, α-Methylitaconsäure, cis-β-Methylglutaconsäure, trans-α-Methylglutaconsäure, Propiolsäure oder Zimtsäure enthalten.

c) Vorteilhaft ist es ebenfalls, wenn das Vorbehandlungsmittel Stoffe enthält, welche die Haarquellung hemmen. Hierfür kommen insbesondere Alkali- oder Erdalkalichloride oder -sulfate in Betracht. Es ist jedoch darauf zu achten, dass das Vorbehandlungsmittel bei Verwendung von Calciumsalzen nicht gleichzeitig Alginsäure oder Alginate enthalten darf.

d) Als pflegenden Stoff kann das Vorbehandlungsmittel zusätzlich beispielsweise Lanolin, Lecithin, Proteine, Glycerin, Betain, Allantoin, Purcellinöl, Silikonöl, Walrat, Fettalkohole, Wollwachs, Paraffinöl, Bienenwachs, niedrigsiedende Isoparaffine oder Silikonöle sowie ausserdem Harnstoff, Eiweiss-Fettsäurekondensate oder aber nichtionische oder amphotere kapilaraktive Substanzen enthalten.

Selbstverständlich kann das Vorbehandlungsmittel gegebenenfalls darüber hinaus übliche kosmetische Zusätze, wie zum Beispiel Farbstoffe, Pigmente, Parfümöle, Emulgatoren und andere, enthalten. Der pH-Wert des Vorbehandlungsmittels ist vorzugsweise 2 bis 6.

Bei dem erfindungsgemässen Verfahren wird menschliches Haar, das einen 3 bis 8 cm langen unverformten Haarnachwuchs aufweist, je nach Haarfülle, zunächst mit 25–50 g eines vorstehend beschriebenen Vorbehandlungsmittels gleichmässig befeuchtet. Das Vorbehandlungsmittel kann in beliebiger flüssiger wässriger Form, beispielsweise als klare, gefärbte oder trübe Lösung, als flüssige Emulsion oder als flüssige Dispersion vorliegen. Das Haar wird anschliessend in Strähnen abgeteilt und auf Wickler gewickelt. Der Durchmesser der Wickler beträgt, je nachdem, ob eine Dauerwellung oder eine Haarentkräuselung durchgeführt wird, entweder 5 bis 13 Millimeter oder mehr. Auf die Oberfläche der Wickler werden insgesamt 40–60 g eines ansonsten üblichen flüssigen oder angedickten creme- oder gelförmigen Dauerverformungsmittels aufgetragen, das jedoch, je nach der Zusammensetzung des Vorbehandlungsmittels, entweder alkalisch ist, falls das Vorbehandlungsmittel einen Stoff enthält, der im alkalischen Bereich ein Gel oder eine Ausfällung ergibt oder aber einen Stoff enthält, der beim Kontakt mit dem Vorbehandlungsmittel aus den anderen, vorstehend geschilderten Gründen ein Gel oder eine Ausfällung ergibt.

Die bei dem hier beschriebenen Verfahren verwendbaren Dauerverformungsmittel sind solche auf der Basis üblicher Reduktionsmittel, wie zum Beispiel Sulfit oder bestimmter Mercaptoverbindungen, insbesondere Salze oder Derivate der Thioglykolsäure wie Ammoniumthioglykolat sowie weiterhin Ammoniumthiolactat, Glycerinmonothioglykolat oder Cystein und seine Derivate. Diese Dauerverformungsmittel enthalten die reduzierenden Verbindungen in den für eine Haarverformung üblichen Mengen, beispielsweise die Ammoniumsalze der Thioglykolsäure oder der Thiomilchsäure, in einer Konzentration von 2 bis 12 Gew.%. Der pH-Wert der alkalischen Dauerverformungsmittel liegt im allgemeinen bei 7,5 bis 11, wobei die Einstellung mit Ammoniak, Monoethanolamin, Ammoniumcarbonat und/oder Ammoniumhydrogencarbonat erfolgt. Bei sauer (zum Beispiel auf pH = 6,5 bis 6,9) eingestellten Dauerverformungsmitteln wird als Reduktionsmittel vorzugsweise Natrium- oder Ammoniumsulfit in einer Konzentration von 3 bis 8 Gew.% (berechnet als $SO_2$) verwendet. Diese üblichen Dauerverformungsmittel können, falls sie alkalisch (pH = 7,5–11) eingestellt sind, direkt in Kombination mit einem Vorbehandlungsmittel eingesetzt werden, das einen Stoff enthält, der im alkalischen Medium ein Gel oder eine Ausfällung ergibt. Falls das Verformungsmittel gemäss dem hier beschriebenen Verfahren einen Stoff enthält, der nur in Kombination mit einem bestimmten anderen Stoff ein Gel oder eine Ausfällung bildet, so wird einer üblichen Dauerverformungsmittelbasis von beliebigem pH-Wert im Bereich 5 bis 11 dieser bestimmte andere Stoff zugesetzt. Die entsprechenden möglichen Kombinationen von Stoffen wurden bereits vorstehend angegeben.

Nach einer für die dauerhafte Verformung des Haares ausreichenden Einwirkungszeit, welche je nach der Haarbeschaffenheit, dem pH-Wert und der Verformungswirksamkeit des Dauerverformungsmittels sowie in Abhängigkeit von der Anwendungstemperatur 5 bis 25 Minuten beträgt, spült man das Dauerverformungsmittel mit Wasser von den Wicklern herunter. Anschliessend werden die gewickelten Haare in üblicher Weise oxidativ fixiert. Die Fixierung dient dazu, einen Überschuss des Verformungsmittels zu neutralisieren und das erweichte Haar wieder zu härten. Die erfindungsgemäss einsetzbaren Oxidationsmittel sind nicht speziell begrenzt; es kann jedes beliebige, bisher dafür verwendete Oxidationsmittel angewendet werden. Beispiele für solche handelsüblichen Oxidationsmittel sind Kaliumbromat, Natriumbromat, Natriumperborat, Hydrogenperoxid und Harnstoffperoxid. Die Konzentration der Oxidationsmittel ist gemäss den Härtungsbedingungen, wie Temperatur und Zeit, variabel. Normalerweise werden in den wässrigen Fixiermitteln die Oxidationsmittel in einer Konzentration von 0,5 bis etwa 10,0 Gew.% verwendet. Das Oxidationsmittel ist zusammen mit anderen bekannten Additiva verwendbar.

Die bei dem erfindungsgemässen Verfahren verwendbaren Dauerverformungsmittel und Fixiermittel können selbstverständlich darüberhinaus in Dauerverformungsmitteln und Fixiermitteln übliche Zusätze, wie zum Beispiel Haarkonditionierungsmittel, Haarpflegemittel, Farbstoffe, Parfümöle und andere enthalten.

Schliesslich werden die Wickler entfernt, das Fixiermittel aus dem Haar ausgespült und das Haar in üblicher Weise weiterbehandelt. In der Regel wird das Haar im Anschluss an eine Dauerverformung zur Wasserwelle gelegt. Im Falle einer Haarentkräuselung kann auch so verfahren werden, dass man das Fixiermittel bei gewickeltem Haar abspült und das Haar anschliessend, ohne abzuwickeln, direkt am Wickler trocknet. Weiterhin ist es auch möglich, das entkräuselte Haar nach dem Entfernen der Wickler und dem Abspülen des Fixiermittels unmittelbar trockenzuföhnen.

Die nachfolgenden Beispiele sollen den Gegenstand der Erfindung näher erläutern.

DAUERWELLUNG

Beispiel 1

Kombination-Stoff, der in alkalischer Lösung ein Gel bildet/alkalisches Dauerwellmittel

Ein dauergewelltes Haar von insgesamt 16 cm Länge, dessen Haarnachwuchs in einer Länge von 3 cm glatt nachgewachsen ist, wird mit einem Shampoo gewaschen, mit Wasser ausgespült und mit einem Handtuch gut abfrottiert. Anschliessend wird das Haar mit 30 g eines Vorbehandlungsmittels der Zusammensetzung

12,0 g einer 30%-igen wässrigen Dispersion eines Copolymerisates aus Methacrylsäu-

re und Acrylsäureethylester (Handelsprodukt ROHAGIT SD 15 der Firma Röhm
GmbH, Darmstadt)

1,0 g　Betainmonohydrat
1,5 g　Eiweissfettsäurekondensat, 28%-ige
　　　　wässrige Lösung (Handelsprodukt Lame-
　　　　pon S der Chemischen Fabrik Grünau
　　　　GmbH, Deutschland)
0,5 g　Polyethylenglycol-Sorbitanmonolaurat
85,0 g　Wasser
──────
100,0 g

vom pH = 5,8 gründlich befeuchtet. Das befeuchtete Haar wird in Strähnen aufgeteilt und auf Dauerwellwickler von 8 Millimeter Durchmesser gewickelt. Auf die Wickler werden 50 g eines Dauerwellpräparates der nachfolgenden Zusammensetzung A oder B aufgetragen:

A)　23,6 g　Ammoniumthioglykolat, 50%-ige wäss-
　　　　　　rige Lösung
　　　6,4 g　Ammoniumhydrogencarbonat
　　　1,0 g　Ammoniumcarbonat
　　　0,4 g　Octylphenol, mit 20 Mol Ethylenoxid
　　　　　　oxethyliert
　　　2,0 g　Dimethyldiallylammoniumchlorid-
　　　　　　Homopolymerisat, 40%-ige wässrige
　　　　　　Lösung
　　　0,4 g　Parfümöl
　　　66,2 g　Wasser
　　　──────
　　　100,0 g

Diese Dauerwellflüssigkeit hat einen pH-Wert
von 8,7.

B)　20,0 g　Ammoniumthioglykolat, 50%-ige wäss-
　　　　　　rige Lösung
　　　4,0 g　Ammoniak, 25%-ige wässrige Lösung
　　　6,0 g　Cetylstearylalkohol
　　　1,0 g　Natriumlaurylsulfat
　　　1,0 g　Parfümöl
　　　68,0 g　Wasser
　　　──────
　　　100,0 g

Diese Dauerwellcreme hat einen pH-Wert von
9,6.

Nach einer Einwirkungszeit von 15 Minuten wird
das gewickelte Haar gründlich mit lauwarmem
Wasser gespült. Überschüssiges Wasser wird mit
einem Handtuch oder einer Papierserviette entfernt. Anschliessend werden 50 g eines Fixiermittels der Zusammensetzung

4,0 g　Hydrogenperoxid, 50%-ige wässrige Lö-
　　　　sung
0,2 g　Phosphorsäure, 85%-ig
1,0 g　Zitronensäure
94,8 g　Wasser
──────
100,0 g

das einen pH-Wert von 2,2 aufweist, auf das gewikkelte Haar aufgetragen.

Nach einer Einwirkungszeit von 5 Minuten werden die Haare abgewickelt, das Fixiermittel durch
Spülen mit Wasser entfernt und das Haar in üblicher Weise zur Wasserwelle gelegt und getrocknet. Es wird ein vom Haaransatz bis zur Haarspitze gleichmässig gekraustes Haar erhalten, wobei
das bereits vorher gekrauste Haar nicht in die
Verformung mit einbezogen wurde und daher chemisch nicht erneut geschädigt ist.

Beispiel 2

Man führt die Dauerwellung des Haarnachwuchses wie in Beispiel 1 beschrieben durch, verwendet jedoch ein Vorbehandlungsmittel der folgenden Zusammensetzung:

15,0 g　einer 28%-igen wässrigen Dispersion ei-
　　　　nes Copolymerisates aus Acrylsäure und
　　　　Acrylsäureethylester (Handelsprodukt
　　　　Primal ASE 60 der Firma Rohm & Haas
　　　　Co. Inc., USA)
10,0 g　Glycerin
1,0 g　Milchsäure, 90%-ig
0,5 g　d, L-Methionin
1,0 g　L-Glutaminsäure
1,0 g　1,4-Nonylphenol, mit 10 Mol Ethylenoxid
　　　　oxethyliert
0,5 g　Parfümöl
71,0 g　Wasser
──────
100,0 g

Der pH-Wert dieser flüssigen Dispersion beträgt
2,6.

Beispiel 3

Man führt die Dauerwellung des Haarnachwuchses wie in Beispiel 1 beschrieben durch, verwendet jedoch ein Vorbehandlungsmittel der folgenden Zusammensetzung:

2,0 g　Chitosan mit 90% freien Aminogruppen
1,0 g　Milchsäure, 90%-ig
2,0 g　Natriumbromat
95,0 g　Wasser
──────
100,0 g

Der pH-Wert dieser Lösung beträgt 5,1.

Beispiel 4

Man führt die Dauerwellung des Haarnachwuchses wie in Beispiel 1 beschrieben durch, verwendet jedoch ein Vorbehandlungsmittel der folgenden Zusammensetzung:

5,0 g　Aluminiumsulfat, $Al_2(SO_4)_3$
1,5 g　Cetylstearylalkohol
0,2 g　Natriumlaurylsulfat
0,5 g　Polyethylenglykol-Sorbitanmonolaurat
0,3 g　Parfümöl
92,5 g　Wasser
──────
100,0 g

Der pH-Wert dieser flüssigen Emulsion ist 3.

Beispiel 5

Man führt die Dauerwellung des Haarnachwuchses wie in Beispiel 1 beschrieben durch, verwendet jedoch ein Vorbehandlungsmittel der folgenden Zusammensetzung:

3,0 g  Ammoniumaluminiumsulfat
       $NH_4Al(SO_4)_2 \cdot 12H_2O$
2,0 g  Trimethylcetylammoniumchlorid,
       25%-ige wässrige Lösung
1,5 g  Natriumchlorid
1,0 g  Glycin
0,3 g  1,4-Nonylphenol, mit 10 Mol Ethylenoxid
       oxethyliert
0,2 g  Parfümöl
92,0 g  Wasser
―――――
100,0 g

Der pH-Wert dieser klaren Lösung beträgt 3,5.

Beispiel 6
   Kombination-Vorbehandlungsmittel mit anionischem Polymer/Dauerwellmittel mit kationischem Polymer
   Bereits dauergewellte Haare von 12 cm Länge, die noch eine ausreichende Krause zeigen, deren Haarnachwuchs jedoch in einer Länge von 4 cm glatt nachgewachsen ist, werden gewaschen, gespült und zur Handtuchtrockene abgefrottiert. Anschliessend wird das Haar mit 30 g eines Vorbehandlungsmittels der Zusammensetzung

6,0 g  60%-ige Lösung eines Mischpolymerisates aus Vinylacetat, Crotonsäure und Acrylsäure in Wasser/Isopropanol (1:4)
       [anionisches Polymer]
3,0 g  Glycerin
1,0 g  Natriumbromat
0,3 g  Ammoniak, 25%-ige wässrige Lösung
89,7 g  Wasser
―――――
100,0 g

vom pH = 7,2 gründlich befeuchtet. Das befeuchtete Haar wird in Strähnen aufgeteilt und auf Dauerwellwickler von 6 Millimeter Durchmesser gewickelt. Auf die Wickler werden anschliessend 50 g eines Dauerwellpräparates mit einem Gehalt an einem kationischen Harz zur Zusammensetzung A (aus Beispiel 1) oder C aufgetragen.

C)  2,0 g  einer 50%-igen wässrigen Lösung eines Copolymerisates aus 80% Vinylpyrrolidon/20% Dimethylaminoethylmethacrylat, teilweise quaternisiert mit Dimethylsulfat (mittleres Molekulargewicht = 100 000)
            [kationisches Polymer]
   24,6 g  Ammoniumthiolactat, 50%-ige wässrige Lösung
    4,0 g  Ammoniak, 25%-ige wässrige Lösung
    0,5 g  1,4-Nonylphenol, mit 10 Mol Ethylenoxyd oxethyliert
    0,2 g  Parfümöl
   68,7 g  Wasser
   ―――――
   100,0 g

Dieses Dauerwellpräparat hat einen pH-Wert von 9,6.
   Nach einer Einwirkungszeit von 12 Minuten wird das gewickelte Haar gründlich mit lauwarmem Wasser gespült. Das überschüssige Wasser wird mit einem Handtuch entfernt. Anschliessend werden 50 g eines üblichen Fixiermittels auf der Basis von Hydrogenperoxid, zum Beispiel eine 2%-ige wässrige $H_2O_2$-Lösung, auf das gewickelte Haar aufgetragen. Nach einer Einwirkungszeit von 3 Minuten wird das Haar abgewickelt, mit weiteren 30 g des Fixiermittels befeuchtet und nach 2 Minuten gründlich mit Wasser gespült. Schliesslich wird das Haar in üblicher Weise zur Wasserwelle gelegt und getrocknet.

Beispiel 7
   Kombination-Vorbehandlungsmittel mit kationischem Polymer/Dauerwellmittel mit anionischem Polymer
   Es wird wie in Beispiel 6 beschrieben verfahren, jedoch verwendet man ein Vorbehandlungsmittel der Zusammensetzung

7,5 g  Kaliumaluminiumsulfat,
       $KAl(SO_4)_2 \cdot 12 H_2O$
3,5 g  Dimethyldiallylammoniumchlorid-Homopolymerisat, mittleres Molekulargewicht = 500 000, 40%-ige wässrige Lösung
       [kationisches Polymer]
1,0 g  Maleinsäure
1,0 g  1,4-Nonylphenol, mit 10 Mol Ethylenoxid oxethyliert
0,2 g  Allantoin
0,3 g  Parfümöl
86,5 g  Wasser
―――――
100,0 g

(Der pH-Wert dieses flüssigen Vorbehandlungsmittels beträgt 2,0).

und ein Dauerwellmittel der Zusammensetzung

3,5 g  60%-ige Lösung eines Copolymerisates aus Vinylacetat, Crotonsäure und Acrylsäure in Wasser/Isopropanol (1:4)
       [anionisches Polymer]
13,0 g  Ammoniumthioglykolat, 50%-ige wässrige Lösung
2,0 g  Ammoniumcarbonat, $(NH_4)_2CO_3$
1,0 g  Ammoniumhydrogencarbonat $NH_4HCO_3$
0,5 g  Octylphenol, mit 20 Mol Ethylenoxid oxethyliert
0,3 g  Parfümöl
79,7 g  Wasser
―――――
100,0 g

Dieses Dauerwellmittel hat einen pH-Wert von 8,8.

ENTKRÄUSELUNG
   Prinzipiell kann bei der Haarentkräuselung analog den Beispielen 1 bis 7 verfahren werden, wobei jedoch Wickler mit einem Durchmesser von über 20 Millimeter zu verwenden sind. Ein weiteres Beispiel ist nachfolgend beschrieben.

Beispiel 8
   Durch eine 3 Monate zurückliegende Haarentkräuselungsbehandlung geglättetes 15 cm langes Haar, dessen Haarnachwuchs in einer Länge von 4 cm nachgewachsen und daher stark gekraust ist, wird mit 30 g eines Vorbehandlungsmittels der Zusammensetzung

| | |
|---|---|
| 5,0 g | Aluminiumsulfat, $Al_2(SO_4)_3$ |
| 1,5 g | Cetylstearylalkohol |
| 0,2 g | Natriumlaurylsulfat |
| 0,5 g | Polyethylenglykol-Sorbitanmonolaurat |
| 0,3 g | Parfümöl |
| 92,5 g | Wasser |
| 100,0 g | |

die als flüssige Emulsion vorliegt und einen pH-Wert von 3 aufweist, gleichmässig befeuchtet. Anschliessend wird das Haar auf Wickler von 24 mm Durchmesser gewickelt. Insgesamt 50 g eines Dauerverformungsmittels in Cremeform der Zusammensetzung B (aus Beispiel 1) werden auf die Wickler aufgetragen. Nach einer Einwirkungszeit von 20 Minuten wird das gewickelte Haar mit Wasser gespült und sodann mit 50 g eines Fixiermittels der Zusammensetzung

| | |
|---|---|
| 8,0 g | Natriumbromat, $NaBrO_3$ |
| 2,0 g | einer 50%-igen wässrigen Lösung eines Copolymerisates aus 80% Vinylpyrrolidon/20% Dimethylaminoethylmethacrylat, teilweise mit Dimethylsulfat quaternisiert (mittleres Molekulargewicht = 100 000) |
| 90,0 g | Wasser |
| 100,0 g | |

dessen pH-Wert 6,8 beträgt, behandelt. Nach einer Einwirkungszeit von 10 Minuten werden die Wickler entfernt. Schliesslich wird das Haar gründlich mit Wasser gespült und trockengefönt. Das Haar ist nach dieser Behandlung unter Schonung der Haarstruktur vom Ansatz bis zur Spitze gleichmässig geglättet.

**Patentansprüche**

1. Verfahren zur selektiven Dauerverformung des Haarnachwuchses, bei dem man das Haar zunächst mit einem flüssigen wässrigen Vorbehandlungsmittel behandelt, auf Wickler wickelt, mit einem wässrigen keratinreduzierenden Dauerverformungsmittel auf der Basis von Sulfit oder Mercaptoverbindungen behandelt, spült, oxidativ fixiert und in üblicher Weise weiterbehandelt, dadurch gekennzeichnet, dass man als Vorbehandlungsmittel eine flüssige wässrige Zusammensetzung verwendet, welche mindestens ein physiologisch verträgliches organisches Salz, anorganisches Salz, natürliches Polycarbonsäurepolymer oder synthetisches Polycarbonsäurepolymer enthält, das bei einem pH-Wert von 2 bis 7 in wässriger Lösung löslich oder dispergierbar ist und bei einem pH-Wert von 7,5 bis 11 ein Gel oder eine Ausfällung ergibt, und sodann ein Dauerverformungsmittel mit einem pH-Wert von 7,5 bis 11 verwendet.

2. Verfahren nach Anspruch 1, dadurch gekennzeichnet, dass man als natürliches Polycarbonsäurepolymer Alginsäure oder als synthetisches Polycarbonsäurepolymer ein physiologisch verträgliches hochpolymeres Homopolymer der Acrylsäure oder der Methacrylsäure verwendet.

3. Verfahren nach Anspruch 1, dadurch gekennzeichnet, dass man als synthetisches Polycarbonsäurepolymer ein hochpolymeres Co- bzw. Mischpolymerisat aus Acrylsäure und/oder Methacrylsäure mit Acrylsäure- oder Methacrylsäureestern, Acrylsäure- oder Methacrylsäureamiden, Acrylsäure- oder Methacrylsäureimiden, Crotonsäure, Vinylacetat, Styrol oder anderen Vinyl- oder Allylderivaten, wobei mindestens 25 Mol-% des Polymerisates aus Carbonsäuregruppen enthaltenden Einheiten bestehen, verwendet.

4. Verfahren nach Anspruch 1, dadurch gekennzeichnet, dass man als synthetisches Polycarbonsäurepolymer ein hochmolekulares Copolymerisat aus Acrylsäure/Methacrylsäure oder Acrylsäure/Acrylsäureester oder Acrylsäure/Methacrylsäureester oder Methacrylsäure/Acrylsäureester oder Methacrylsäure/Methacrylsäureester, wobei die genannten Ester insbesondere der Methyl-, Ethyl-, Propyl- oder Butylester sind, verwendet.

5. Verfahren nach Anspruch 1, dadurch gekennzeichnet, dass man als physiologisch verträgliches organisches oder anorganisches Salz Aluminiumacetat, Ammoniumaluminiumsulfat oder Ammoniumcernitrat verwendet.

6. Verfahren nach Anspruch 1, dadurch gekennzeichnet, dass man als organisches Salz ein wasserlösliches physiologisch verträgliches Salz des Chitosans verwendet.

7. Verfahren nach Anspruch 1 bis 6, dadurch gekennzeichnet, dass man das organische Salz, anorganische Salz, natürliche Polycarbonsäurepolymer oder synthetische Polycarbonsäurepolymer in einer Menge von 1 bis 10 Gew.-% verwendet.

8. Verfahren zur selektiven Dauerverformung des Haarnachwuchses, bei dem man das Haar zunächst mit einem flüssigen wässrigen Vorbehandlungsmittel behandelt, auf Wickler wickelt, mit einem wässrigen keratinreduzierenden Dauerverformungsmittel auf der Basis von Sulfit oder Mercaptoverbindungen behandelt, spült, oxidativ fixiert und in üblicher Weise weiterbehandelt, dadurch gekennzeichnet, dass man als Vorbehandlungsmittel eine wässrige Zusammensetzung verwendet, welche Alginsäure oder ein Alkalialginat enthält und sodann ein Dauerverformungsmittel verwendet, das ein wasserlösliches Salz des Calciums enthält.

9. Verfahren zur selektiven Dauerverformung des Haarnachwuchses, bei dem man das Haar zunächst mit einem flüssigen wässrigen Vorbehandlungsmittel behandelt, auf Wickler wickelt, mit einem wässrigen keratinreduzierenden Dauerverformungsmittel auf der Basis von Sulfit oder Mercaptoverbindungen behandelt, spült, oxidativ fixiert und in üblicher Weise weiterbehandelt, dadurch gekennzeichnet, dass man als Vorbehandlungsmittel eine flüssige wässrige Zusammensetzung verwendet, welche ein anionisches Polymer enthält und sodann ein Dauerverformungsmittel verwendet, welches ein kationisches Polymer enthält.

10. Verfahren zur selektiven Dauerverformung des Haarnachwuchses, bei dem man das Haar zunächst mit einem flüssigen wässrigen Vorbehandlungsmittel behandelt, auf Wickler wickelt, mit einem wässrigen keratinreduzierenden Dauerverformungsmittel auf der Basis von Sulfit oder Mercaptoverbindungen behandelt, spült, oxidativ fixiert und in üblicher Weise weiterbehandelt, dadurch gekennzeichnet, dass man als Vorbehandlungsmittel eine flüssige wässrige Zusammensetzung verwendet, welche ein kationisches Polymer enthält und sodann ein Dauerverformungsmittel verwendet, welches ein anionisches Polymer enthält.

11. Verfahren nach Anspruch 9 und 10, dadurch gekennzeichnet, dass man als anionisches Polymer ein Copolymerisat aus Vinylacetat, Crotonsäure und Acrylsäure verwendet.

12. Verfahren nach Anspruch 9 bis 11, dadurch gekennzeichnet, dass man als kationisches Polymer ein Copolymerisat aus Vinylpyrrolidon und Dimethylaminoethylmethacrylat, welches mit Dimethylsulfat quaternisiert ist, oder ein Dimethyldiallylammoniumchlorid-Homopolymerisat verwendet.

13. Verfahren nach Anspruch 9 bis 12, dadurch gekennzeichnet, dass man das kationische Polymer und das anionische Polymer im Vorbehandlungsmittel beziehungsweise im Dauerverformungsmittel jeweils in einer Konzentration von 0,5 bis 3,0 Gew.% verwendet.

14. Verfahren nach Anspruch 1 bis 13, dadurch gekennzeichnet, dass man ein Vorbehandlungsmittel verwendet, welches zusätzlich mindestens eine der schwachen Säuren Zitronensäure, Weinsäure, Milchsäure, Phosphorsäure, Essigsäure oder saure Phosphate oder neutrale oder saure Aminosäuren enthält.

15. Verfahren nach Anspruch 1 bis 14, dadurch gekennzeichnet, dass man ein Vorbehandlungsmittel verwendet, welches zusätzlich Natriumbromat, Aconitsäure, Acetylendicarbonsäure, Ethylendicarbonsäure, Ethylmaleinsäure, α-Ethylcrotonsäure, i-Amylmaleinsäure, Angelicasäure, n-Butylfumarsäure, n- und i-Butylmaleinsäure, Citraconsäure, Crotonsäure, Fumarsäure, trans-Glutaconsäure, Isopropylmaleinsäure, Itaconsäure, Maleinsäure, Mesaconsäure, α-Methylitaconsäure, cis-β-Methylglutaconsäure, trans-α-Methylglutaconsäure, Propiolsäure oder Zimtsäure enthält.

16. Verfahren nach Anspruch 1 bis 15, dadurch gekennzeichnet, dass man ein Vorbehandlungsmittel verwendet, welches zusätzlich Lanolin, Lecithin, Glycerin, Betain, Allantoin, Purcellinöl, Silikonöl, Walrat, Fettalkohole, Wollwachs, Paraffinöl, Bienenwachs, niedrigsiedende Isoparaffine oder Silikonöle enthält.

## Claims

1. Method for the selective permanent shaping of newly-growing hair wherein the hair is initially treated with a liquid aqueous pretreatment agent, wound onto curlers, treated with an aqueous keratin reducing permanent shaping agent based on sulphite or mercapto-compounds, rinsed, oxidatively fixed and then further treated in conventional manner, characterised in that the pretreatment agent used is a liquid aqueous composition containing at least one physiologically tolerable organic salt, inorganic salt, natural polycarboxylic acid polymer or synthetic polycarboxylic acid polymer and which, at a pH value of 2 to 7, is soluble or dispersible in an aqueous solution while at a pH value of 7.5 to 11 it produces a gel or a precipitate, after which a permanent shaping agent with a pH value of 7.5 to 11 is used.

2. Method according to Claim 1, characterised in that the natural polycarboxylic acid polymer used is alginic acid while the synthetic polycarboxylic acid polymer used is a physiologically tolerable high-polymeric homopolymer of acrylic acid or methacrylic acid.

3. Method according to Claim 1, characterised in that a highly polymeric co- or mixed polymerisate of acrylic acid and/or methacrylic acid with acrylic acid or methacrylic acid esters, acrylic acid or methacrylic acid amides, acrylic acid or methacrylic acid imides, crotonic acid, vinyl acetate, styrene or other vinyl or allyl derivatives can be used as the synthetic polycarboxylic acid polymer, at least 25% mole of the polymerisate consisting of units containing carboxylic acid groups.

4. Method according to Claim 1, characterised in that a high molecular copolymer derived from acrylic acid/methacrylic acid or acrylic acid/acrylic acid ester or acrylic acid/methacrylic acid ester or methacrylic acid/acrylic acid ester or methacrylic acid/methacrylic acid ester is used as the synthetic polycarboxylic acid polymer, the said esters being in particular the methyl, ethyl, propyl or butyl esters.

5. Method according to Claim 1, characterised in that aluminium acetate, ammonium aluminium sulphate or ammonium cerium nitrate is used as the physiologically tolerable oganic or inorganic salt.

6. Method according to Claim 1, characterised in that a water soluble physiologically tolerable salt of chitosan is used as the organic salt.

7. Method according to Claim 1 to 6, characterised in that the organic salt, inorganic salt, natural polycarboxylic acid polymer or synthetic polycarboxylic acid polymer is used in a quantity of 1 to 10% by weight.

8. Method for the selective permanent shaping of newly-growing hair in which the hair is first treated with a liquid aqueous pretreatment agent, wound onto curlers, treated with an aqueous keratin reducing permanent shaping agent based on sulphite or mercaptocompounds, rinsed, oxidatively fixed and further treated in the usual manner, characterised in that an aqueous composition is used as the pretreatment agent, which contains alginic acid or an alkali alginate, followed by a permanent shaping agent which contains a water soluble salt of calcium.

9. Method for the selective permanent shaping of newly-growing hair in which the hair is first treated with a liquid aqueous pretreatment agent, wound onto curlers, treated with an aqueous keratin reducing permanent shaping agent based on sulphite or mercaptocompounds, rinsed, oxidatively fixed and further treated in the usual manner, characterised in that a liquid aqueous composition is used as the pretreatment agent, which contains an anionic polymer and in that a permanent shaping agent is then used which contains a cationic polymer.

10. Method for the selective permanent shaping of newly-growing hair in which the hair is first treated with a liquid aqueous pretreatment agent, wound onto curlers, treated with an aqueous keratin reducing permanent shaping agent based on sulphite or mercaptocompounds, rinsed, oxidatively fixed and further treated in the usual manner, characterised in that a liquid aqueous composition is used as the pretreatment agent, which contains a cationic polymer and in that a permanent shaping agent is then used which contains an anionic polymer.

11. Method according to Claim 9 and 10, characterised in that a copolymerisate from vinyl acetate, crotonic acid and acrylic is used as the anionic polymer.

12. Method according to Claim 9 to 11, characterised in that a copolymerisate from vinyl pyrrolidone and dimethyl amino ethyl methacrylate is used as the cationic polymer, being quaternised with dimethyl sulphate, or in that a dimethyl diallyl ammonium chloride homopolymerisate is used.

13. Method according to Claim 9 to 12, characterised in that the cationic polymer and the anionic polymer in the pretreatment agent or in the permanent shaping agent are in each case used in a concentration of 0.5 to 3% by weight.

14. Method according to Claim 1 to 13, characterised in that a pretreatment agent is used which additionally contains at least one of the weak acids citric acid, tartaric acid, lactic acid, phosphoric acid, acetic acid or acid phosphates or neutral or acid amino acids.

15. Method according to Claim 1 to 14, characterised in that a pretreatment agent is used which in addition contains sodium bromate, aconitic acid, acetylene dicarboxylic acid, ethylene dicarboxylic acid, ethyl maleic acid, α-ethyl crotonic acid, i-amyl maleic acid, angelic acid, n-butyl fumaric acid, n- and i-butyl maleic acid, citraconic acid, crotonic acid, fumaric acid, trans-glutaconic acid, isopropyl maleic acid, itaconic acid, maleic acid, mesaconic acid, α-methyl itaconic acid, cis-β-methyl glutaconic acid, trans-α-methyl glutaconic acid, propiolic acid or cinnamic acid.

16. Method according to Claim 1 to 15, characterised in that a pretreatment agent is used which contains in additional lanolin, lecithin, glycerin, betain, allantoin, purcellin oil, silicon oil, spermaceti, fatty alcohols, wool wax, paraffin oil, beeswax, low-boiling isoparaffins or silicon oils.

**Revendications**

1. Procédé de déformation permanente sélective des repousses de cheveux, au cours duquel on traite d'abord les cheveux par un agent de prétraitement aqueux liquide, on les enroule sur des rouleaux, on les traite par un agent de déformation permanente aqueux réducteur de la kératine à base de sulfite ou de composés mercapto, on les rince, on les fixe par voie oxydante et on continue à les traiter de façon usuelle, caractérisé en ce qu'on utilise comme agent de prétraitement une composition aqueuse liquide qui contient au moins un sel organique, un sel inorganique, un polymère d'acide polycarboxylique naturel ou un polymère d'acide polycarboxylique synthétique physiologiquement compatible, qui, avec un pH de 2 à 7, est soluble ou dispersible dans une solution aqueuse, et qui, avec un pH de 7,5 à 11 donne un gel ou un précipité, et on utilise ensuite un agent de déformation permanente ayant un pH de 7,5 à 11.

2. Procédé selon la revendication 1, caractérisé en ce qu'on utilise comme polymère d'acide polycarboxylique naturel l'acide alginique ou comme polymère d'acide carboxylique synthétique un homopolymère fortement polymérisé, physiologiquement compatible, de l'acide acrylique ou de l'acide méthacrylique.

3. Procédé selon la revendication 1, caractérisé en ce qu'on utilise comme polymère d'acide polycarboxylique synthétique un copolymère ou un produit de copolymérisation fortement polymérisé d'acide acrylique et/ou d'acide méthacrylique avec des esters acryliques ou méthacryliques, des amides acryliques ou méthacryliques, des imides acryliques ou méthacryliques, de l'acide crotonique, de l'acétate de vinyle, du styrène ou d'autres dérivés vinyliques ou allyliques, une proportion molaire d'au moins 25% du polymérisat étant constituée par des unités contenant des groupes d'acides carboxyliques.

4. Procédé selon la revendication 1, caractérisé en ce qu'on utilise comme polymère d'acide polycarboxylique synthétique un copolymérisat de poids moléculaire élevé d'acide acrylique et d'acide méthacrylique, ou d'acide acrylique et d'ester acrylique, ou d'acide acrylique et d'ester méthacrylique, ou d'acide méthacrylique et d'ester acrylique, ou d'acide méthacrylique et d'ester méthacrylique, les esters utilisés étant notamment les esters méthyliques, éthyliques propyliques ou butyliques.

5. Procédé selon la revendication 1, caractérisé en ce qu'on utilise comme sel organique ou inorganique physiologiquement compatible l'acétate d'aluminium, le sulfate d'ammonium-aluminium ou le cérinitrate d'ammonium.

6. Procédé selon la revendication 1, caractérisé en ce qu'on utilise comme sel organique un sel de chitosane soluble dans l'eau et physiologiquement compatible.

7. Procédé selon les revendications 1 à 6, caractérisé en ce qu'on utilise le sel organique, le sel inorganique, le polymère d'acide polycarboxylique naturel ou le polymère d'acide polycarboxylique synthétique dans une proportion de 1 à 10% en poids.

8. Procédé de déformation permanente sélective des repousses des cheveux, dans lequel on traite d'abord les cheveux avec un agent de traitement liquide aqueux, on les enroule sur des rouleaux, on les traite avec un agent de déformation permanente aqueux réducteur de la kératine à base de sulfite ou de composés mercapto, on les rince, on les fixe par voie oxydante et on continue le traitement de façon usuelle, caractérisé en ce qu'on utilise comme agent de prétraitement une composition aqueuse qui contient de l'acide alginique ou un alginate alcalin et on utilise ensuite un agent de déformation permanente, qui contient un sel de calcium soluble dans l'eau.

9. Procédé de déformation permanente sélective des repousses des cheveux, dans lequel on traite d'abord les cheveux avec un agent de prétraitement liquide aqueux, on les enroule sur des rouleaux, on les traite avec un agent de déformation permanente aqueux réducteur de la kératine à base de sulfite ou de composés mercapto, on les rince, on les fixe par voie oxydante et on continue le traitement de la façon usuelle, caractérisé en ce qu'on utilise comme agent de prétraitement une composition aqueuse liquide, qui contient un polymère anionique et on utilise ensuite un agent de déformation permanente qui contient un polymère cationique.

10. Procédé de déformation permanente sélective des repousses des cheveux, dans lequel on traite d'abord les cheveux avec un agent de prétraitement liquide aqueux, on les enroule sur des rouleaux, on les traite avec un agent de déformation permanente aqueux réducteur de la kératine à base de sulfite ou de composés mercapto, on les rince, on les fixe par voie oxydante, et on continue le traitement de la façon usuelle, caractérisé en ce qu'on utilise comme agent de prétraitement une composition liquide aqueuse qui contient un polymère cationique et on utilise ensuite un agent de déformation permanente qui contient un polymère anionique.

11. Procédé selon la revendication 9 ou 10, caractérisé en ce qu'on utilise comme polymère anionique un copolymère d'acétate de vinyle, d'acide crotonique et d'acide acrylique.

12. Procédé selon les revendications 9 à 11, caractérisé en ce qu'on utilise comme polymère cationique, un copolymère de vinylpyrrolidone et de méthacrylate de diméthylaminoéthyle, formant avec le diméthylsulfate un composé quaternaire, ou bien un homopolymère de chlorure de diméthyl-diallyl-ammonium.

13. Procédé selon les revendications 9 à 12, caractérisé en ce qu'on utilise le polymère cationique ou le polymère anionique dans l'agent de prétraitement, et respectivement dans l'agent de déformation permanente, à une concentration de 0,5 à 3% en poids.

14. Procédé selon les revendications 1 à 13, caractérisé en ce qu'on utilise un agent de prétraitement qui contient, en plus, au moins l'un des acides faibles suivants: l'acide citrique, un acide tartrique, l'acide lactique, l'acide phosphorique, l'acide acétique ou des phosphates acides, ou des amino-acides neutres ou acides.

15. Procédé selon les revendications 1 à 14, caractérisé en ce qu'on utilise un agent de prétraitement qui contient en plus du bromate de sodium, de l'acide aconitique, l'acide acétylène-dicarboxylique, l'acide éthylène-dicarboxylique, l'acide éthylmaléique, l'acide α-éthyl-crotonique, l'acide i-amylmaléique, l'acide angélique, l'acide n-butylfumarique, l'acide n- et i-butylmaléique, l'acide citroconique, l'acide crotonique, l'acide fumarique, l'acide trans-glutaconique, l'acide isopropylmaléique, l'acide itaconique, l'acide maléique, l'acide mésaconique, l'acide α-méthylitaconique, l'acide cis-β-méthylglutaconique, l'acide trans-α-méthylglutaconique, l'acide propanoïque ou l'acide cinnamique.

16. Procédé selon les revendications 1 à 15, caractérisé en ce qu'on utilise un agent de prétraitement qui contient, en plus, de la lanoline, de la lécithine, du glycérol, de la bétaïne, de l'allantoïne, de l'huile de purcelline, l'huile silicone, du blanc de baleine, des alcools gras, de la cire de laine, de l'huile de paraffine, de la cire d'abeille, des isoparaffines ou des huiles silicones à bas point d'ébullition.